# EUROPEAN PATENT SPECIFICATION

(11) **EP 1 069 441 B1**
(45) Date of publication and mention of the grant of the patent: **14.12.2005**
(21) Application number: 00305904.5
(22) Date of filing: 12.07.2000
(51) Int. Cl.: G02B 5/02, G02B 5/08

(54) **Reflectors for UV radiation source**
Reflektoren für eine ultraviolette Strahlungsquelle
Réflecteurs pour une source de radiation ultraviolette

(30) Priority: 13.07.1999 US 143608 P; 29.02.2000 US 515191
(43) Date of publication of application: 17.01.2001
(73) Proprietor: Johnson & Johnson Vision Care, Inc., Jacksonville, FL 32256 (US)
(72) Inventor: Ebel, James A., Jacksonville, FL 32256 (US); Kimble, Allan W., Jacksonville, FL 32217 (US); Enns, John B., Jacksonville, FL 32257 (US)
(74) Representative: Mercer, Christopher Paul

(56) References cited:
- WO-A-99/08137
- WO-A-99/21913

## Description

### FIELD OF THE INVENTION

This invention relates to a new reflector for a radiation source which delivers ultraviolet radiation.

### BACKGROUND OF THE INVENTION

US Patent 5,786,598 discloses the broad concept that a flash lamp system might be used for deactivating microorganisms in containers including a polyolefin container with a foil backing that contains a contact lens and a preservative fluid. Although the patent discloses the idea of using a flash lamp system to sterilize contact lenses in a preserved solution in a container, there are no conditions defined to accomplish sterility, nor examples provided which show that sterility can be accomplished.

U.S. Patents 5,034,235 and 4,871,559 disclose the use of intermittent pulses of very intense, very short duration pulses of light to inactivate microorganisms on the surface of food products, and suggests that the method can be used for containers, medical devices, and food products in packages.

Efforts have been made to use a flash lamp system for sterilization of contact lenses in containers; however, to date no system has been developed that will consistently achieve the sterility required to provide a high confidence level in the system. In experimentation with a commercially available system sterility was not achieved for all microorganisms under the normal operating conditions. To increase the radiation intensity to the containers it was necessary to increase the voltage applied to the flash lamps; however, this shortened the lifetime of the lamps, and also damaged the container and product materials. Further, anytime a lamp was changed, which was required more frequently due to the shortened life of the lamp, the lamp to lamp variations in radiation intensity resulted in variations in the sterility level that was achieved. For the sterilization of a single-use contact lens, the USFDA requires a minimum sterility assurance level (SAL) of 10⁻⁶ (number of microorganisms per container). A sterility assurance level of 10⁻⁶ is the probability of having 1 non-sterile container out of one million containers. Therefore, there still remains a need for a way to deliver high energy UV radiation uniformly to a product for sterilization using a flash lamp system that is repeatable and capable of commercial use.

### SUMMARY OF THE INVENTION

This invention provides a reflector as defined in claim 1 for a high energy radiation system as defined in claim 19 wherein said reflector at least partially encompasses a radiation source comprising a reflective material having a diffuse reflective surface which reflects greater than 50 % of the radiation from 240-280nm which impinges upon it. The reflector of this invention provides uniform UV radiation to a target area or volume. Not only is the radiation more uniform, but the diffuse reflectors are able to reflect more of the radiation from a lamp which impinges upon the reflectors as compared to specular reflectors. The uniform UV radiation allows the lamps to be used at lower voltages to deliver a minimum quantity of radiation everywhere within a target volume which extends their useful lives. The variability from lamp to lamp is decreased by the diffuse reflector.

This invention also provides for a reflector or reflectors which are elliptical in shape. If multiple elliptically shaped reflectors are used to form a reflective cavity, preferably where substantially all or all of the surfaces of the cavity are diffuse reflective surfaces, the bases of the reflectors where the reflectors intersect to form the volume to be filled by the target are sized to the target, thereby providing minimal dead space. Dead space is where radiation could otherwise pass by the target without impinging on the target. The design of the reflectors maximizes the reflection of the radiation from the lamp to the target.

The new reflectors make it possible to use a radiation system, preferably a pulsed flash lamp system having these reflectors to sterilize products including pharmaceutical, medical, and cosmetic products, and can be used in an in-line mode in the manufacture of these products. The reflectors disclosed herein which provide uniform radiation to a target can also be used for photopolymerization, surface treatments and laser applications.

### DESCRIPTION OF THE FIGURES

The invention will be described with reference to the following figures:
Figure 1 shows a cross-section of a radiation system having two reflectors of this invention;
Figure 2 shows a cross-section of a radiation system having one reflector of this invention;
Figure 3 shows a contour of the radiation energies in a target area using a prior art reflector.
Figure 4 shows a contour of the radiation energies in a target area using the same reflector used to generate the contour shown in Fig. 3, except that the surface of the reflector had a diffuse coating applied to it.
Figure 5 shows a contour of the radiation energies in a target area using a reflector having the shape shown in Fig. 1 and a diffuse coating.
Figure 6 is a graph which shows the Q Factor for a radiation system having a cavity formed using two of the reflectors used to generate the contour shown in Fig. 3.
Figure 7 is a graph which shows the Q Factor for a radiation system having a cavity formed using two of the reflectors used to generate the contour shown in Fig. 4.
Figure 8 is a graph which shows the Q Factor for a radiation system having a cavity formed using two of the reflectors used to generate the contour shown in Fig. 5.

### DESCRIPTION OF THE INVENTION

The term "ultraviolet radiation" or "UV radiation" means radiation having a wavelength or wavelengths from 200 to 400nm. If a range is specified following the term "ultraviolet radiation" or "UV radiation", a narrower range of radiation is meant within the 200 to 400nm range. Further, the range specified, unless otherwise stated, means radiation having a wavelength or wavelengths within the range.

The term "broad spectrum of radiation" means radiation having at least a majority of the wavelengths from 20 to 1100nm wherein at least a portion of the radiation is UV radiation.

The phrase "to direct radiation towards a target" means to send radiation towards a target by reflection, transmission or reflective-emission. The directed radiation may reach the target directly, and/or indirectly minus any amounts which are attenuated intentionally or unintentionally. The "target" is the medical device, container, or surface at which the radiation is directed.

This invention provides reflectors for use for a high energy radiation source. A high energy radiation source that can be used with the reflectors of this invention includes discrete or continuum producing, incoherent lamps, such as flash lamps, arc lamps, lasers (continuous or non-continuous), deuterium lamps, or continuous wave light sources, e.g. xenon gas or mercury vapor light sources. The UV radiation sources are high energy, that is, they generate greater than 0.1 J/cm² per pulse for a flash lamp or 20 watts/cm² for a continuous radiation source, preferably of which at least 1 percent of the radiation is from 240 to 280nm. The presently preferred UV radiation source is a flash lamp system, having any number of lamps, e.g. one to six lamps, which produces at least 1 J/cm² broad spectrum radiation (100 - 3000nm) per flash of which at least 10 mJ/cm² per flash is UV radiation. The preferred application of this invention is in a flash lamp system for the sterilization of contact lenses (target).

EP-A-1033138 discloses more details of the method of sterilizing contact lenses. The radiation from 240 to 280nm is the most effective range for the sterilization of microorganisms, with many references indicating that 254nm is the peak of that range.

The reflector comprises a reflective material and a diffuse reflective surface. The reflector reflects greater than 50 %, more preferably greater than 75 %, and most preferably greater than 90 % of the radiation from 240 to 280nm which impinges upon it. Many of the reflectors of this invention using the materials described herein reflect greater than 95% of the radiation from 240 to 280nm. For some embodiments the reflective material reflects greater than 50 %, more preferably greater than 75 %, and most preferably greater than 90 % of the radiation from 250 to 270nm which impinges upon it. The ranges 240 to 280nm, and 250 to 270nm will be referred to as the desired ranges, the reflector may or may not reflect additional radiation outside the ranges specified, but at a minimum will reflect the specified percentages of the radiation within the desired ranges. The amount of reflected radiation may include radiation which is absorbed by the reflector and re-emitted at different wavelengths within the desired ranges. It is preferred that the reflector reflects at least a portion of all the wavelengths of desired radiation which impinges upon it. The reflective materials may not reflect all the wavelengths within the desired ranges at a single percentage, so certain reflective materials will be better suited for some applications than others. Mixtures of the reflective materials can be used to achieve improved reflection at certain or all of the wavelengths in the desired ranges.

The reflectors of this invention provide a Quality Factor, ("Q") greater than 1.7, preferably greater than 2 most preferably greater than 3 . The Quality Factor is defined as the ratio of the total energy measured in the target area from all the radiation sources and their reflectors configured in a closed cavity divided by the summation of the energy from each lamp and reflector measured individually in the target area or volume of an open cavity. For the preferred embodiment comprising two reflectors and two lamps, the Q is greater than 3, preferably greater than 4, and most preferably greater than 5.

The reflective materials which are part of the reflector reflects radiation from a radiation source to a target. The target can be any material which is to be effected by the radiation for the purpose of, for example, sterilization, photoactivation, surface treatments, photopolymerization, etc. The target can be, for example, products, particularly a medical product, polymers, monomers, laser medium, and dyes. The preferred target is a medical product for sterilization. The reflector of this invention is shaped to direct the desired radiation toward the target. Preferably one or more of the reflectors of this invention are used in a radiation system comprising one or more lamps. Preferably the reflector or reflectors substantially encompass or at least partially encompass the radiation source or sources of the radiation. Preferably the reflectors encompass at least 180° from the centerline of the lamp. Additionally, the reflector or reflectors preferably encompass the target too. It is preferred that the reflector or reflectors preferably form a cavity, tunnel, hollow sphere, or chamber encompassing the target area or volume which is sized to be substantially the size and shape of the target, such that the amount of radiation that can pass from one reflector to another reflector or from one surface to another surface of the reflector located on opposite sides of the target without going through or being absorbed by the target is minimized. Preferably less than 50 percent more preferably less than 25 percent and most preferably less than 10 percent of the total radiation produced by each radiation source will be able to pass by the target without going through or being absorbed by the target. The target area or volume is located at the focal point or plane of the reflector(s). The reflector reflects the desired radiation towards the target either directly or indirectly, that is, the desired radiation may impinge an apparatus before striking the target, e.g. another surface of the reflector or reflectors, mirrors, fiber optics, or the like.

The diffuse reflectors of this invention deliver a uniform amount of radiation, at least of the desired radiation, to a target area or volume. A uniform amount of radiation means that the variation in the levels of the energy within the target area and/or volume is less than 8 mJ/cm², more preferably less than 6 mJ/cm², most preferably less than 5 mJ/cm². The variation in the levels of the energy within the target area or volume is less than 15 percent, more preferably less than 10 percent, most preferably less than 5 percent. Therefore, for two radiation systems having equivalent radiation sources encompassed by reflector(s) to form a treatment cavity having a target area or volume, the only difference being the types of reflectors, the system using the diffuse reflector(s) will typically provide at least an equivalent average amount of radiation to a target area or volume, and a more uniform amount of radiation in the target area or volume.

The diffuse reflectors also provide radiation at a broader array of angles with the benefit, in the preferred embodiment, that there is a smaller chance that microorganisms will escape the radiation. When specular reflectors are used the angle of incidence of the radiation is not as varied increasing the likelihood that microorganisms may be shielded from the radiation by other microorganisms, or by refracting, diffracting, or reflecting elements within the package. The angle of incidence to a target area or volume for a system having one or more specular reflectors is from one-half degree to 6 degrees. For a system having one or more reflectors of this invention, the angles of incidence comprise angles from 40 to 180 degrees. The greater variance in the angle of incidence in the radiation to the target which is more lethal to the microorganisms, and provide for a larger contrast ratio. The contrast ratio is the ratio of the intensity of radiation in an unobstructed (empty) target area or volume to the intensity of radiation for an obstructed target area or volume. An obstructed target area or volume is one in which there is one or more elements present in the target area or volume which diminish intensity due to absorption, reflection, refraction, defraction, or scatter. Examples of such elements include lenses, packaging, microorganisms, bubbles, surface geometries, etc.. The radiation can be measured using the monitoring system described in EP-A-99310248.2

The obstructed measurement of radiation can be made by placing a sensor within the packaging. The reflectors of this invention provide a contrast ratio of less than 1.5 in the target area or volume. This can be compared to a specular reflector which would provide a contrast ratio of greater than 10.

The preferred reflective materials for the diffuse reflectors of this invention include, but are not limited to, alkaline metal compounds (oxides and halides), heavy metal oxides (e.g. barium), divalent metal oxides (e.g. magnesium), and polyvalent metal oxides (e.g. ytterbium or aluminum). Reflective materials can also be selected according to the following formula MₐO_{b}X_{c}H_{d} wherein M is a single metal or a mix of metals, preferably a rare earth metal, O is oxygen, X is a heteroatom such as sulfur, nitrogen and phosphorous or the like, and H is a halide, preferably fluorine, a is 1 to 20, preferably 1 to 12, b is 0 to 20, preferably 0 to 12, c is 0 to 20, preferably 0 to 12, and d is 0 to 20, preferably 0 to 12, with the proviso that at least b, c or d is at least 1. These materials need to be of sufficient purity such that the levels of impurities do not degrade the reflector performance. Preferably the materials are more than 99.9 % pure, more preferably more than 99.99 % pure. Examples of useful reflective materials are listed in Table 1. Included in Table 1 are the mean percent reflectivities of the reflective materials. The percent reflectivities were determined by packing a dry powder sample of solid material into a cuvette, and putting the cuvette into a spectrophotometer having an integrating sphere which measured the radiation reflected from the sample.

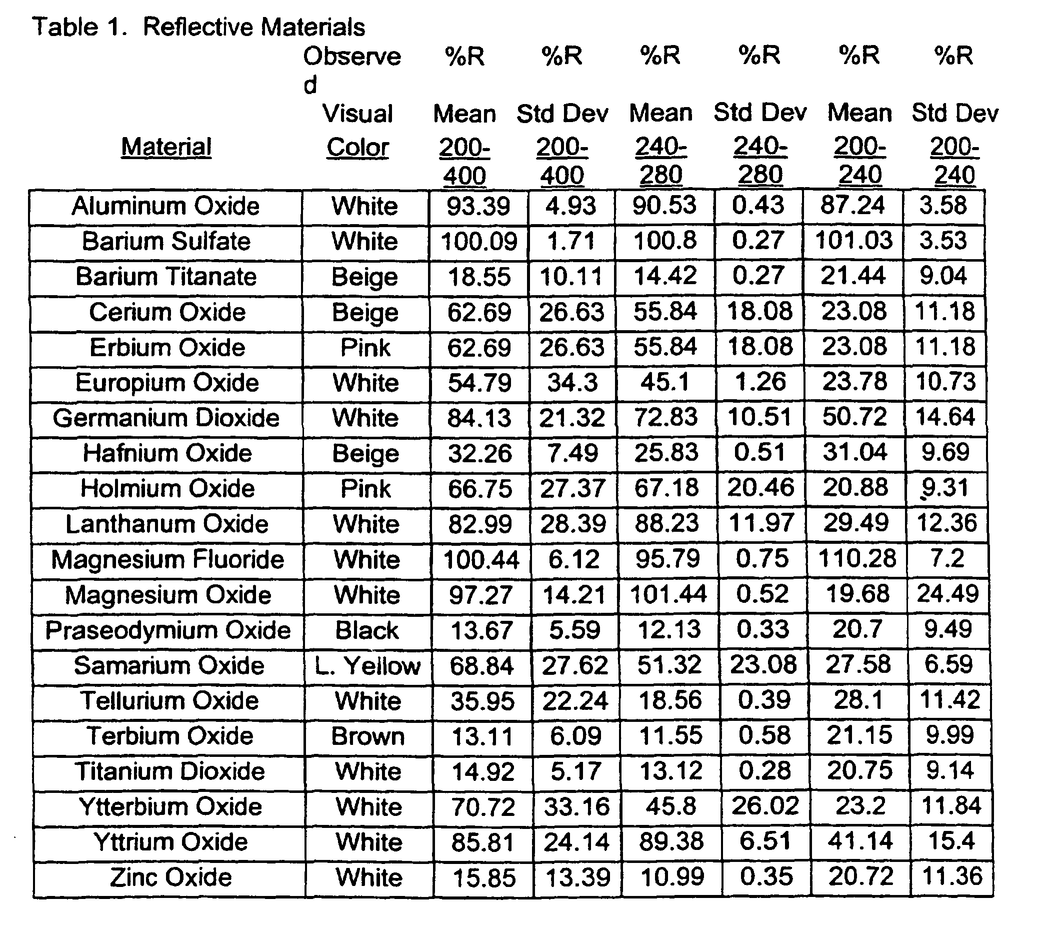

In the formula for the reflective materials, when a is 1 to 6 and b is 2 to 11, and c and d are 0 then the reflective materials is a metal oxide, such as calcium oxide (CaO) and hafnium oxide (HfO₂), lanthanum oxide (La₂O₃), terbium oxide (Tb₄O₇), and barium titanate (BaTiO₃). An example of reflective material for which a is 1 and d is 2 and b and c are 0 is magnesium fluoride (MgF₂). Additional examples of reflective materials include magnesium oxide (MgO), aluminum oxide (Al₂O₃) barium oxide (BaO), barium titanate (BaTiO₃), holmium oxide (Ho₂O₃), calcium oxide (CaO), lanthanum oxide (La₂O₃), germanium oxide (GeO₂), tellurium oxide (TeO₂), europium oxide (Eu₂O₃), erbium oxide (Er₂O₃), neodymium oxide (Nd₂O₃), samarium oxide (Sm₂O₃), ytterbium oxide (Yb₂O₃), yttrium oxide (Y₂O₃), magnesium fluoride (MgF2), barium sulfate (BaSO4), and dysprosium oxide (Dy₂O₃). Other examples include refractory oxides of other rare earths, rare earth halides and metallic combination oxides. The preferred reflective materials are magnesium oxide, magnesium fluoride, aluminum oxide, barium sulfate, lanthanum oxide, yttrium oxide, and ytterbium oxide, and the most preferred are magnesium oxide, magnesium fluoride, aluminum oxide and barium sulfate.

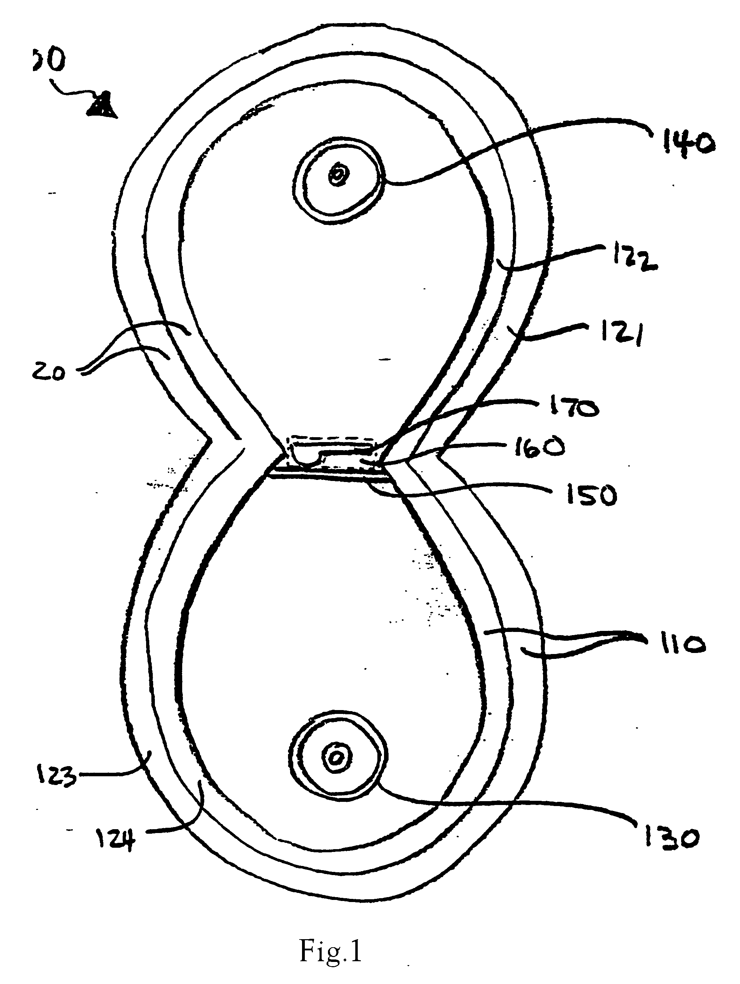

The preferred shape for the reflectors is elliptical. For a single elliptical shaped reflector the target area or volume and the location of the lamp are preferably at the foci of the ellipse. For a radiation system having more than one reflector, which preferably intersect to form a cavity, the target area or volume is preferably located at or encompasses the foci of the ellipses, and the one or more lamps are located at the opposite foci of the ellipses. In the preferred design, having two elliptically shaped reflectors, the foci of each reflector opposite the lamps is at a different location within the target volume. In the preferred design (shown in Fig. 1) the foci of the lower reflector is at the top of the target volume and the foci of the top reflector is at the bottom of the target volume.

Fig. 1 shows two diffuse reflectors 110 and 120 of this invention for use in a radiation system 100, e.g. a flash lamp system 100. (More information on flash lamp systems can be found in US Patents 4,464,336; 5,034,235; and 4,871,559, incorporated herein by reference.) The radiation system comprises reflectors 110 and 120, and flash lamps 130 and 140. Reflector 110 substantially encompasses flash lamp 130. Reflector 120 substantially encompasses flash lamp 140. Flash lamps 130, 140 are located at one foci of each reflector 120, 130, respectively. Reflectors 110 and 120 encompass the target volume 160 (shown in dashed lines) in which the target 170 is placed. The second foci (not shown) of reflectors 120, 130 are within the target volume. As shown, the reflectors are shaped to provide a target volume which allows minimal radiation to pass from one reflector to another reflector without passing through the target. The target 170 is a contact lens container containing a contact lens (not shown) and solution (not shown). The target 170 is held in place by a target support 150 which defines the bottom of the target volume 160. The target support 150 is a transparent glass, crystalline material, quartz plate or the like 150. In Fig. 1, the flash lamp system 100 is shown having a vertical configuration; however, the system can be rotated by any number of degrees; however, the target support 150 might have to be modified or changed to for example, a hook, conveyor, or hollow block to accommodate the target 170. Alternatively, there can be any number of reflectors to direct the radiation at the target. Preferably the reflectors are designed to form a closed cavity, which can be opened via a door or the like to place the target within the cavity, the treatment with radiation can occur when the cavity is closed, and then the cavity can be opened or otherwise accessed to remove the target after treatment. When the cavity is closed, the cavity is light-tight.

The reflectors are shown having the same size and shape; however, they can differ if desired. As shown, the reflectors 110 and 120 each comprise a reflector support 121, 123 and a reflective coating 122, 124. The reflective coatings 122, 124 can be made of any reflective material which provides a diffuse reflective layer. The reflective coatings 122, 134 are shown as a single layer, but the coatings 122, 124 can comprise multiple layers of various reflective materials, if desired.

The reflective coatings 122, 124 can be the same or different and can be applied by painting, spraying, dipping, casting, conversion coating, gel coating, etching, chemical vapor depositing, sputtering, plasma spraying, laserdeposition, or chemical or mechanical bonding, e.g. by adhesives of a film comprising the attenuating materials to the reflector support 121, 123. The preferred method of applying the reflective coatings is to paint or spray reflective materials onto the reflector support 121, 123. To paint or spray them onto the support 121, 123, an aqueous or non-aqueous suspension is formed preferably comprising reflective material and binder. Useful binders are polymeric, inorganic or sol-gels, more preferably inorganic or sol gel, and most preferably inorganic. The preferred suspension comprises 0.1 to 50 % by weight binder, 0.1 to 99.9 % by weight reflective material, and 0.1 to 90 % by weight carrier. The carrier is a liquid used to form a dilution of the reflective materials and binder to apply the coating. Examples of useful carriers are water, alcohols, alkanes, freons, and the like, most preferably water.

Examples of polymeric binders useful in making coatings comprising reflective materials are polyvinyl alcohols, cyanoacrylates, acrylics, and silicones. Presently the polymeric binders are limited in their use, because they tend to degrade in the high energy UV radiation. Examples of inorganic binders useful in making coatings comprising reflective materials are sodium silicate, low-temperature sintered glasses, alkali oxide silicates, such as sodium, potassium and lithium silicates. Examples of sol gel binder precursors useful in making coatings comprising reflective materials are aluminum tert butoxide, sodium silicate, tetraethylorthosilicate (TEOS), metal isopropoxides, dysprosium ethylhexano-diisopropoxide in isopropanol, dysprosium 2-ethylhexanoate in hexane, dysprosium isopropoxide in toluene-isopropanol, dysprosium 2-methoxyethoxide in 2-methoxyethanol, erbium ethylhexano-diisopropoxide in isopropanol, erbium 2-ethylhexanoate in hexane, erbium isopropoxide in toluene -isopropanol, holmium ethylhexano-diisopropoxide in isopropanol, holmium isopropoxide in toluene -isopropanol, holmium 2-methoxyethoxide in 2-methoxyethanol, lanthanum acetate, lanthanum 2-ethylhexanoate in hexane, lanthanum isopropoxide, lanthanum 2-methoxyethoxide in 2-methoxyethanol, magnesium ethoxide in ethanol, magnesium methoxide in methanol, magnesium 2-methoxyethoxide in 2-methoxyethanol, neodymium ethylhexano-diisopropoxide in isopropanol, neodymium 2-ethylhexanoate in hexane, neodymium isopropoxide in toluene - isopropanol, neodymium 2-methoxyethoxide in 2-methoxyethanol, samarium ethylhexano-monoisopropoxide in toluene isopropanol, samarium 2-ethylhexanoate in hexane, samarium isopropoxide in toluene -isopropanol, samarium 2-methoxyethoxide in 2-methoxyethanol, ytterbium isopropoxide in toluene-isopropanol, ytterbium 2-methoxyethoxide in 2-methoxyethanol, yttrium ethylhexano-diisopropoxide in toluene-isopropanol, yttrium ethylhexano-monoisopropoxide in toluene-isopropanol. The preferred sol gel precursors are erbium ethylhexano-diisopropoxide in isopropanol, erbium 2-ethylhexanoate in hexane, erbium isopropoxide in toluene -isopropanol, holmium ethylhexano-diisopropoxide in isopropanol, holmium isopropoxide in toluene -isopropanol, holmium 2-methoxyethoxide in 2-methoxyethanol, lanthanum acetate, lanthanum 2-ethylhexanoate in hexane, lanthanum isopropoxide, lanthanum 2-methoxyethoxide in 2-methoxyethanol, magnesium ethoxide in ethanol, magnesium methoxide in methanol, magnesium 2-methoxyethoxide in 2-methoxyethanol, samarium ethylhexano-monoisopropoxide in toluene isopropanol, samarium 2-ethylhexanoate in hexane, samarium isopropoxide in toluene -isopropanol, samarium 2-methoxyethoxide in 2-methoxyethanol, ytterbium isopropoxide in toluene-isopropanol, ytterbium 2-methoxyethoxide in 2-methoxyethanol, yttrium ethylhexano-diisopropoxide in toluene-isopropanol, yttrium ethylhexano-monoisopropoxide in toluene-isopropanol. The more preferred sol gel precursors are lanthanum acetate, lanthanum 2-ethylhexanoate in hexane, lanthanum isopropoxide, lanthanum 2-methoxyethoxide in 2-methoxyethanol, ytterbium isopropoxide in toluene-isopropanol, ytterbium 2-methoxyethoxide in 2-methoxyethanol, yttrium ethylhexano-diisopropoxide in toluene-isopropanol, yttrium ethylhexano-monoisopropoxide in toluene-isopropanol.

Some of the binders can be used alone as the reflective materials, particularly the sol gels which can be applied as described above in a suspension or sintered to form a reflective composition, either coating or solid block. Examples of binder precursors which can be used alone as the reflective materials include dysprosium isopropoxide, dysprosium ethylhexano-diisopropoxide in isopropanol, dysprosium 2-ethylhexanoate in hexane, dysprosium isopropoxide in toluene -isopropanol, dysprosium 2-methoxyethoxide in 2-methoxyethanol, erbium ethylhexano-diisopropoxide in isopropanol, erbium 2-ethylhexanoate in hexane, erbium isopropoxide in toluene -isopropanol, holmium ethylhexano-diisopropoxide in isopropanol, holmium isopropoxide in toluene -isopropanol, holmium 2-methoxyethoxide in 2-methoxyethanol, Lanthanum acetate, Lanthanum 2-ethylhexanoate in hexane, Lanthanum isopropoxide, Lanthanum 2-methoxyethoxide in 2-methoxyethanol, Magnesium ethoxide in ethanol, Magnesium methoxide in methanol, Magnesium 2-methoxyethoxide in 2-methoxyethanol, Neodymium ethylhexano-diisopropoxide in isopropanol, Neodymium 2-ethylhexanoate in hexane, Neodymium isopropoxide in toluene -isopropanol, Neodymium 2-methoxyethoxide in 2-methoxyethanol, Samarium ethylhexano-monoisopropoxide in toluene isopropanol, Samarium 2-ethylhexanoate in hexane, Samarium isopropoxide in toluene -isopropanol, Samarium 2-methoxyethoxide in 2-methoxyethanol, Ytterbium isopropoxide in toluene-isopropanol, Ytterbium 2-methoxyethoxide in 2-methoxyethanol, yttrium ethylhexano-diisopropoxide in toluene-isopropanol, Yttrium ethylhexano-monoisopropoxide in toluene-isopropanol.

Alternatively, the reflective coatings described above can comprise any of the reflective materials and binders listed above which are formed in the shape of a film and then chemically or mechanically bonded to a reflector support. Alternatively, the reflective materials can be combined with metal oxides or powdered glass and sintered to form films of the reflective materials which can be chemically or mechanically bonded to a reflector support. As stated above, the most preferred reflective materials are barium sulfate, aluminum oxide, magnesium fluoride, and magnesium oxide.

The reflective coatings are preferably applied to form a coating having a thickness from 0.1 to 2500 microns. (A coating greater than 2500 microns is considered a block of the material). The coatings are preferably applied in multiple layers of the same attenuating material(s), preferably using the same coating composition.

The reflector supports 121, 123 can be non-reflective reflector supports or reflective reflector supports which can comprise additional coatings, such as films or foils onto which the one or more reflective coatings described above are applied. Reflective supports can be diffuse or specular. Reflective supports can comprise metal. An example of a reflective reflector support is a metal, such as, solid polished aluminum, which is thick enough to hold its shape, and is bolted or otherwise mounted into place encompassing the lamp, or is a film, e.g. a vapor-deposited specular metal sheet, which is adhered to another part having the desired reflector shape to form a reflective reflector support. The reflective reflector support can also be made from solid blocks comprising reflective materials onto which the reflective coating can be applied. Reflectors comprising solid blocks of the reflective materials will be described below.

Almost any material can be used as a non-reflective reflector support including wood, polymers, metals and ceramics.

In an alternative embodiment, the diffuse reflector of this invention can comprise formed solids comprising the reflective materials. The formed solids can be formed by combining the reflective materials with metal oxides or powdered glass, and sintering them to form the reflector. The reflective materials and metal oxides or powdered glass are sintered in the shape of the reflector. Alternatively, the reflector can be made by combining the reflective materials with binders and forming a formed solid either in the shape of the reflector or not in the shape of the reflector and subsequently machining the formed solid into the shape of the reflector. Alternatively, a glass reflector can be formed by adding the reflective materials as a dopant to the feed stock used to make a quartz, crystalline material, sapphire, or UV radiation-transparent glass in the shape of the reflector.

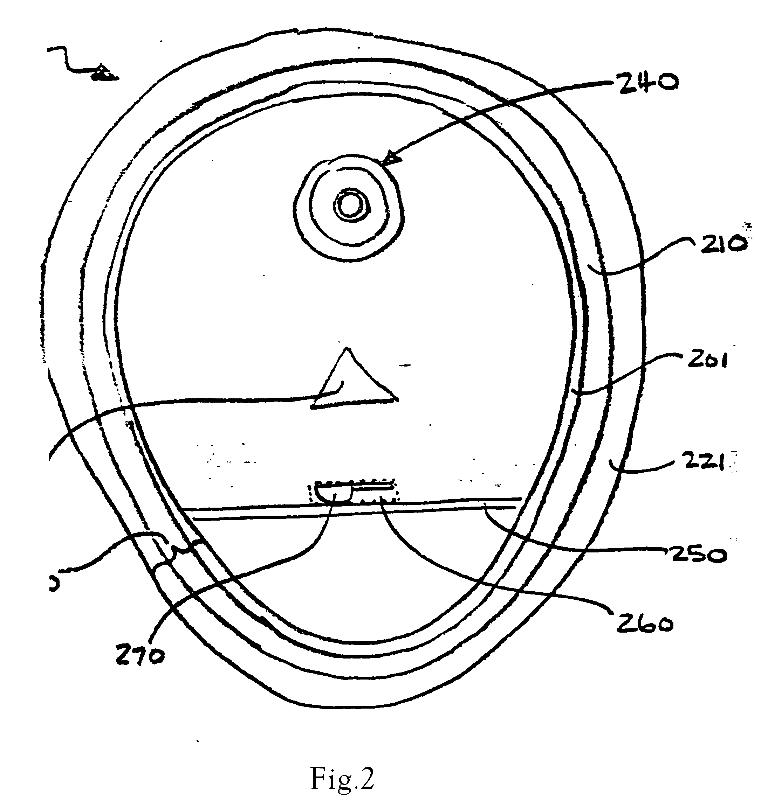

Fig. 2 shows an alternative embodiment of this invention. In Fig. 2 the radiation system shown comprises a single lamp and a single reflector 220. The reflector 220 encompasses both the lamp and the target. The target volume 260 is at the foci, focal point or plane of the reflector (shown in dashes), and is where the target 270 is placed. The target 270 is a contact lens container. The reflector 220 comprises a reflector support 221, a material layer 210 and a transparent support 201. The transparent support 201 is transparent to at least a portion of the radiation which impinges upon it, preferably the desired radiation. The transparent support 201 preferably comprises glass, quartz, sapphire, crystalline material or the like. The reflector support 221 can comprise any of the combinations of reflective or non-reflective supports, or coatings on the supports as described above. The material layer 210 comprises one or more reflective materials, and/or can be any of the compositions comprising reflective materials as described above; however, this embodiment is particularly suited for reflective materials that will not stay in place without the presence of the transparent support 201, such as a packed powder. The material layer 47 preferably has a thickness from 0.1 to 2500 microns.

Also shown in Fig. 2 in the radiation system 200 is an optional reflective blocking element 202. The reflective blocking element 202 can be used to provide more uniform radiation to a target area or volume particularly in a situation where the direct radiation from the radiation source is non-uniform. To prevent the radiation from the radiation source from striking the target directly, the reflective blocking element 202 is placed between the radiation source 240 and the target 270. The reflective blocking element 202 preferably has a simple geometric form, more preferably an optically concentrating form, and most preferably an integral form of the reflecting optics. Examples of useful shapes are a triangle (shown in Fig. 2) and a half circle. It is preferred that the reflective blocking element is a diffuse reflector comprising the reflective materials described herein. The reflective blocking element can comprise any of the reflector compositions described herein. Preferably, the blocking element is sized to occlude any direct radiation from the radiation source to the target.

All the diffuse reflectors described herein can comprise optional attenuating materials as a coating on the reflector, or as an additive to the reflector compositions. Attenuating materials are used to attenuate undesired radiation from the radiation which is produced by the radiation source and eventually reaches the target. Attenuating materials and ways to add the attenuating materials to a radiation system are disclosed and described in concurrently filed European patent application No. claiming priority from USSN 60/143 607 (Attorney's ref: P025029EP).

Any of the attenuating materials and the ways of incorporating the attenuating materials into a radiation system disclosed therein can be used with the reflectors herein. In fact, some of the attenuating materials disclosed in the referenced application are reflective materials useful in this invention. Because some of the reflective materials reflect some wavelengths of radiation and absorb other wavelengths of radiation, careful selection of reflective materials may provide for the attenuation of undesired radiation. For the preferred use of the reflectors of this invention, that is, in a radiation system which sterilizes contact lenses, the radiation from 100 up to 240nm which damages the contact lens polymers is preferably attenuated. It is preferred to attenuate greater than 30 %, more preferably greater than 60 % and most preferably greater than 90 % of the undesired radiation from the radiation to the contact lens polymers. Examples of reflective materials which also attenuate the radiation from 100 up to 240nm are magnesium oxide, lanthanum oxide, ytterbium oxide, and yttrium oxide.

The uniformity in the radiation directed to the target area or volume is greatly improved using a diffuse reflector having any shape compared to a specular reflector having the same shape. In addition the quantity of the radiation can be increased by diffuse reflector or reflectors which at least partially encompass the lamp or lamps, even more preferably at least substantially encompass the lamp(s) and the target, forming a cavity or chamber, preferably a closed cavity in which all the radiation is directed toward the target by the lamps and the reflective surfaces of the cavity. The one or more reflectors can have a cylindrical, polygonal, parabolic or elliptical shape; however, the preferred shape is an elliptical shape. Elliptical reflectors are shown in Figs. 1 and 2. The prior art reflectors are specular and have straight sides which cause a large loss in the radiation, particularly when the radiation hits the straight sides at a small angle of incidence. The preferred radiation system comprises two elliptically-shaped reflectors which have a target volume with minimal space between the reflector cavity and the target to minimize the amount of radiation which passes from one reflector to the other without hitting the target. However, any configuration of one or more diffuse reflectors provide the benefits described herein.

### Example and Comparative Examples

A spectroradiometric monitoring system was used to measure the radiation within the desired range of 240 to 280nm in a target area that two prior art reflectors and a reflector of this invention, individually and in pairs, directed at the target area using a pulsed radiation system. The monitoring system is further described in EP-A-00301248.2.

The monitoring system disclosed in Ebel et al, can measure the spectroradiometric output of each flash. The reflectors were mounted in pairs into the same radiation system, and the spectroradiometric measurements were performed. The reflectors all had the same parabolic shape. The radiation system was a PurePulse PBS1-4 system (manufactured by PurePulse Technologies, Inc., San Diego, CA) which consisted of two flash lamp assemblies connected in series, each of the flash lamp assemblies consisted of a lamp, and a reflector partially encompassing the lamp. The PBS1-4 system consists of a pulse-generator capable of generating a large pulse of energy by virtue of its large capacitance (80-160 µF) and high potential (greater than 6 kV), and control circuits. Both lamps generate a broad spectrum of radiation which includes ultraviolet, infrared and visible light. For two of the three measurements made for this example using the reflectors described below, the radiation from the second lamp and reflector was isolated from the first lamp and reflector (for which the measurements were made) by using an aluminum sheet between the lamp assemblies. The first measurements made were used to generate a contour plot of the radiant energy per flash for one lamp and one reflector (one lamp assembly) in the target area 21 mm from the lamp assembly's protective window.

After generating the contour plot, the radiant energies at one point (where an integrating sphere was placed) 21 mm away from the lamp was measured for one lamp and one of each of the reflectors described below as the voltage to the lamp was varied. The total measured radiant energy from 240 to 280nm was plotted as a function of the voltage. Then, the just-described steps were repeated except that both lamp assemblies, each consisting of a lamp and a reflector described below, were used to measure the total radiant energy from 240 to 280nm at the same point. (The aluminum sheet between the lamp assemblies was removed.) The total measured radiant energy from 240 to 280nm was plotted as a function of the voltage on the same graph as when the radiant energy from only one lamp assembly was measured.

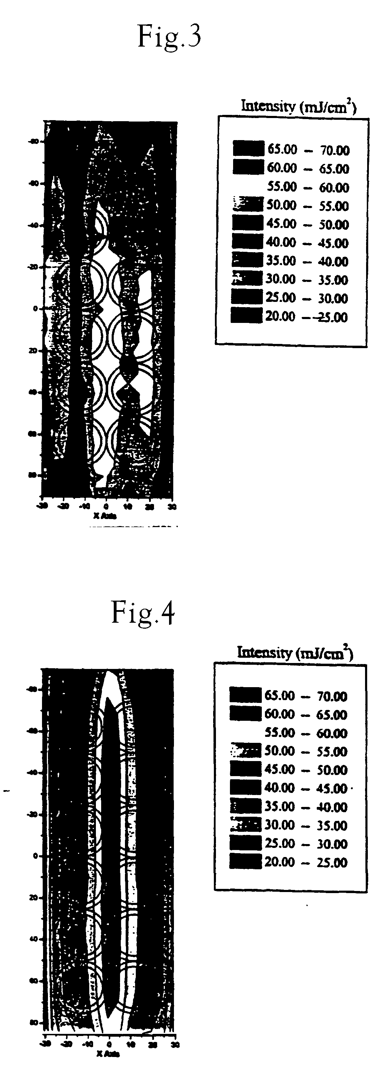

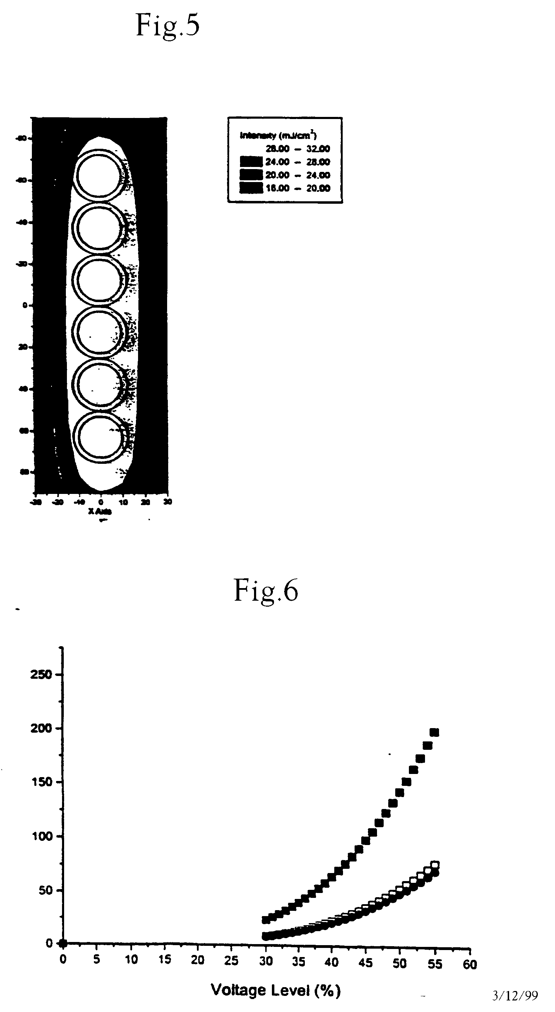

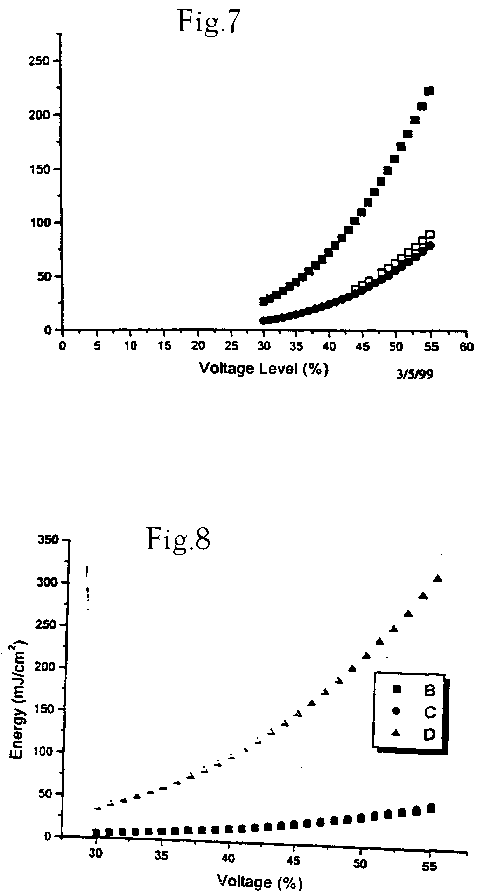

A blocking element was not part of the radiation system.
Prior Art Reflector A was a shaped aluminum reflector having a vapor-deposited layer of aluminum on which was a coating of silicon monoxide for protection, provided by the manufacturer of the PBS1-4 System. A contour plot of the radiant energy using the Prior Art Reflector A is shown in Fig. 3. Fig. 6 shows the a curve of the total energy from 240 to 280nm at different lamp voltages measured for one lamp assembly using the Prior Art Reflector A, and a second curve of the total energy in the cavity formed by both lamp assemblies and having straight sides between the lamp assemblies using the Prior Art Reflector A.
Reflector B was made by modifying the Prior Art Reflector A by adhering a thin sheet of a specular aluminum foil, coated with an antioxidative protective layer onto Prior Art Reflector A. The specular aluminum foil material used is called a Light Sheet. A contour plot of the radiant energy directed into the target area of the lamp using the Prior Art Reflector B is shown in Fig. 4. Fig. 7 shows a curve of the total energy from 240 to 280nm at different lamp voltages measured for one lamp assembly using the Prior Art Reflector B, and a second curve of the total energy in a cavity formed by both lamp assemblies using the Reflector B. Reflector B provides a more uniform level of energy than Prior Art Reflector A.
Reflector C was made by modifying the Prior Art Reflector A by coating it with BaSO₄. The surface of the Prior Art Reflector A was first blasted with small glass beads. A barium sulfate coating composition was prepared by mixing 1 part by weight sodium silicate (binder), 10 parts by weight barium sulfate (reflective material) and 10 parts by weight water (carrier). Twenty layers of the coating composition were sprayed onto the aluminum substrate. Each coating was air dried between coatings. A contour plot of the radiant energy directed into the target area of the lamp using the Reflector C is shown in Fig. 5. Fig. 8 shows the a curve of the total energy from 240 to 280nm at different lamp voltages measured for one lamp assembly using the Reflector C, and a second curve of the total energy in a cavity formed by both lamp assemblies using the Reflector C.

The contour plot of the Reflector C (Fig. 5) shows the increase in uniformity of the energy that the diffuse reflector directs at the target area as compared to the Prior Art Reflector A and Reflector B (Fig. 3 and 4) Greater uniformity decreases the chances that microorganisms can avoid the minimum dose required for sterilization. The circles on the contour plots represent the target, that is, 12 contact lens containers which can be removably-attached to each other. These removably-attached containers are commonly used to configure multi-packs of contact lens containers.

Figure 8, compared to Figure 6, shows that the preferred diffuse reflector of this invention when used to at least partially encompass the lamp(s) and the target area or volume, although more preferably used to form a cavity, more preferably a closed cavity in which the radiation from the lamp(s) can be reflected and re-reflected and directed at the target, provide a higher Q, and higher energy than the same chambers using specular reflectors.

This invention has been described with reference to particular embodiments; however, variations within the scope of the following claims are apparent to those of ordinary skill in the art.

## Claims

1. A reflector (110, 120, 220) for a high energy radiation system comprising a diffuse reflective surface which reflects greater than 50 % of the radiation from 240 to 280nmand has a Quality Factor of greater than 1.7 the Quality Factor being defined as the ratio of the total energy measured in a target area from all the radiation sources and their reflectors configured in a closed cavity divided by the summation of the energy from each lamp and reflector measured individually in the target area or volume of an open cavity, wherein said diffuse reflective surface comprises one or more of aluminum oxide, barium sulfate, barium titanate, barium oxide, calcium oxide, cerium oxide, dysprosium oxide, erbium oxide, europium oxide, germanium dioxide, hafnium oxide, holmium oxide, lanthanum oxide, magnesium fluoride, magnesium oxide, neodymium oxide, praseodymium oxide, samarium oxide, tellurium oxide, terbium oxide, titanium dioxide, ytterbium oxide, yttrium oxide, zinc oxide.

2. The reflector (110, 120, 220) of claim 1 wherein said reflector (110, 120, 220) reflects greater than 75%, preferably greater than 90%, of the radiation from 240 to 280 nm, more preferably greater than 90% of the radiation from 250 to 270nm.

3. The reflector (110, 120, 220) of claim 1 or claim 2 wherein said reflector (110, 120, 220) is elliptical.

4. The reflector (110, 120, 220) of any one of claims 1 to 3 wherein said reflector (110, 120, 220) provides radiation energy to a target area wherein said variation in the energy is less than 8 mJ/cm².

5. The reflector (110, 120, 220) of any one of clams 1 to 4 wherein said reflector provides radiation energy to a target area wherein said variation in the energy is less than 15 percent, preferably less than 10 percent.

6. The reflector (110, 120, 220) of any one of claims 1 to 5 wherein said reflector provides radiation to a target area and said radiation comprises angles of incidence from 40 to 180 degrees.

7. The reflector (110, 120, 220) of any one of claims 1 to 6 wherein said reflector provides a contrast ratio of less than 1.5.

8. The reflector (110, 120, 220) of any one of claims 1 to 7 comprising one or more rare earth, rare earth halide or metallic combination oxide reflective materials.

9. The reflector (110, 120, 220) of any one of claims 1 to 7 comprising one or more calcium oxide, hafnium oxide, lanthanum oxide, terbium oxide, barium titanate, magnesium fluoride, magnesium oxide, aluminium oxide, barium oxide, holmium oxide, germanium oxide, tellurium oxide, europium oxide, erbium oxide, neodymium oxide, samarium oxide, ytterbium oxide, yttrium oxide, barium sulfate or dysproium oxide, preferably magnesium oxide, magnesium fluoride, aluminium oxide, barium sulfate, lanthanum oxide, yttrium oxide or ytterbium oxide, most preferably magnesium oxide, magnesium floride, aluminium oxide, or barium sulfate reflective materials.

10. The reflector (110, 120, 220) of any one of claims 1 to 9 comprising one or more reflective materials which are more than 99.9% pure.

11. The reflector (110, 120, 220) of any one of claims 1 to 8 wherein said reflector comprises a reflective coating or film (122, 134, 210) attached to a support (121, 123, 201).

12. The reflector (110, 120, 220) of claim 11 wherein said reflector comprises a reflective coating (122, 134) which is applied by painting, spraying, dipping, casting, conversion coating, gel coating, etching, chemical vapor depositing, sputtering, plasma spraying or laser depositing.

13. The reflector (110, 120, 220) of claim 11 or claim 12 wherein said reflective coating or film (122, 134, 210) is from 0.1 to 2500 microns in thickness.

14. The reflector (110, 120, 220) of any one of claims 1 to 8 wherein said reflector comprises a packed powder or block of reflective materials.

15. The reflector (110, 120, 220) of any one of claims 1 to 8 wherein said reflector comprises a polyvinyl alcohol, cyanoacrylate, acrylic or silicone material.

16. The reflector (110, 120, 220) of any one of claims 1 to 8 wherein said reflector comprises a sodium silicate, low-temperature sintered glass or alkali oxide silicate material.

17. The reflector (110, 120, 220) of any one of claims 1 to 8 wherein said reflector comprises an aluminum tert butoxide, sodium silicate, tetraethylorthosilicate (TEOS), metal isopropoxide, dysprosium isopropoxide, dysprosium ethylhexano-diisopropoxide in isopropanol, dysprosium 2-ethylhexanoate in hexane, dysprosium isopropoxide in toluene-isopropanol, dysprosium 2-methoxyethoxide in 2-methoxyethanol, erbium ethylhexano-diisopropoxide in isopropanol, erbium 2-ethylhexanoate in hexane, erbium isopropoxide in toluene-isopropanol, holmium ethylhexano-diisopropoxide in isopropanol, holmium isopropoxide in toluene-isopropanol, holmium 2-methoxyethoxide in 2-methoxyethanol, lanthanum acetate, lanthanum 2-ethylhexanoate in hexane, lanthanum isopropoxide, lanthanum 2-methoxyethoxide in 2-methoxyethanol, magnesium ethoxide in ethanol, magnesium methoxide in methanol, magnesium 2-methoxyethoxide in 2-methoxyethanol, neodymium ethylhexano-diisopropoxide in isopropanol, neodymium 2-ethylhexanoate in hexane, neodymium isopropoxide in toluene-isopropanol, neodymium 2-methoxyethoxide in 2-methoxyethanol, samarium ethylhexano-monoisopropoxide in toluene-isopropanol, samarium 2-ethylhexanoate in hexane, samarium isopropoxide in toluene-isopropanol, samarium 2-methoxyethoxide in 2-methoxyethanol, ytterbium isopropoxide in toluene-isopropanol, ytterbium 2-methoxyethoxide in 2-methoxyethanol, yttrium ethylhexano-diisopropoxide in toluene-isopropanol or yttrium ethylhexano-monoisopropoxide in toluene-isopropanol material.

18. A radiation system (100, 200) comprising at least one high energy radiation source (130, 140, 240) and at least one reflector (110, 120, 220) which at least partially encompasses said at least one radiation source (130, 140, 240) wherein said at least one reflector (110, 120, 220) is diffuse and has a Quality Factor of greater than 1.7 the Quality Factor being defined as the ratio of the total energy measured in a target area from all the radiation sources and their reflectors configured in a closed cavity divided by the summation of the energy from each lamp and reflector measured individually in the target area or volume of an open cavity, wherein said diffuse reflective surface comprises one or more of aluminum oxide, barium sulfate, barium titanate, barium oxide, calcium oxide, cerium oxide, dysprosium oxide, erbium oxide, europium oxide, germanium dioxide, hafnium oxide, holmium oxide, lanthanum oxide, magnesium fluoride, magnesium oxide, neodymium oxide, praseodymium oxide, samarium oxide, tellurium oxide, terbium oxide, titanium dioxide, ytterbium oxide, yttrium oxide, zinc oxide.

19. The radiation system (100, 200) of claim 18 wherein said at least one radiation source is a flash lamp (130, 140, 240).

20. The radiation system (100, 200) of claim 18 or claim 19 further comprising multiple radiation sources (130, 140) which are each at least partially encompassed by multiple reflectors (110,120), wherein said multiple reflectors (110, 120) are diffuse.

21. The radiation system (100, 200) of any one of claims 18 to 20 wherein said at least one reflector (110, 120, 220) having a diffuse reflective surface reflects greater than 50% of the radiation from 240 to 280nm.

22. The radiation system (100, 200) of any one of claims 18 to 21 wherein said at least one reflector fully (110, 120, 220) encompasses said at least one radiation source (130, 140, 240) to form a closed cavity.

23. The radiation system (100, 200) of claim 22 wherein all the inside surfaces of said cavity are diffusely reflective and which reflect greater than 50% of the radiation from 240 to 280nm.

24. The radiation system (100, 200) of any one of claims 18 to 23 wherein said at least one reflector (110,120, 220) is elliptically shaped.

25. The radiation system (100, 200) of any one of claims 18 to 24 further comprising a target volume, wherein said target volume comprises a contact lens in a contact lens package.

26. The radiation system (100, 200) of any one of claims 18 to 25 wherein each lamp (130, 140, 240) has at least one reflector and said at least one reflector provides a Quality Factor preferably greater than 2 and most preferably greater than 3.

27. The radiation system (100, 200) of any one of claims 18 to 26 wherein the amount of radiation which can pass from one reflector to another reflector without going through the target is less than 50 percent.

28. The radiation system (100, 200) of any one of claims 18 to 27 further comprising attenuating materials.

29. The radiation system (100, 200) of any one of claims 18 to 28 wherein said at least one reflector comprises a barium sulfate, aluminium oxide, magnesium fluoride or magnesium oxide reflective material.

## Patentansprüche

1. Reflektor (110, 120, 220) für ein Hochenergiestrahlungssystem, umfassend eine diffus reflektierende Oberfläche, die mehr als 50 % der Strahlung von 240 bis 280 nm reflektiert und einen Qualitätsfaktor von mehr als 1,7 hat, wobei der Qualitätsfaktor als das Verhältnis der gesamten Energie gemessen in einem Zielbereich aller Strahlungsquellen und ihrer Reflektoren, die in einem geschlossenen Hohlraum angeordnet sind, geteilt durch die Summe der Energie jeder Lampe und jedes Reflektors, die individuell im Zielbereich oder Volumen eines offenen Hohlraumes gemessen wird, definiert ist,
wobei die diffuse reflektierende Oberfläche eines oder mehrere der folgenden umfaßt: Aluminiumoxid, Bariumsulfat, Bariumtitanat, Bariumoxid, Kalziumoxid, Ceroxid, Dysprosiumoxid, Erbiumoxid, Europiumoxid, Germaniumdioxid, Hafniumoxid, Holmiumoxid, Lanthanoxid, Magnesiumfluorid, Magnesiumoxid, Neodymoxid, Praseodymiumoxid, Samariumoxid, Telluroxid, Terbiumoxid, Titandioxid, Ytterbiumoxid, Yttriumoxid, Zinkoxid.

2. Reflektor (110, 120, 220) nach Anspruch 1, **dadurch gekennzeichnet, daß** der Reflektor (110, 120, 220) mehr als 75 %, vorzugsweise mehr als 90 %, der Strahlung von 240 bis 280 nm reflektiert, noch bevorzugter mehr als 90 % der Strahlung von 250 bis 270 nm.

3. Reflektor (110, 120, 220) nach Anspruch 1 oder Anspruch 2, **dadurch gekennzeichnet, daß** der Reflektor (110, 120, 220) elliptisch ist.

4. Reflektor (110, 120, 220) nach einem der Ansprüche 1 bis 3, **dadurch gekennzeichnet, daß** der Reflektor (110, 120, 220) Strahlungsenergie zu einem Zielbereich liefert, wobei die Variation der Energie geringer als 8 mJ/cm² ist.

5. Reflektor (110, 120, 220) von einem der Ansprüche 1 bis 4, **dadurch gekennzeichnet, daß** der Reflektor Strahlungsenergie zu einem Zielbereich liefert, wobei die Variation der Energie geringer als 15 % ist, vorzugsweise geringer als 10 %.

6. Reflektor (110, 120, 220) nach einem der Ansprüche 1 bis 5, **dadurch gekennzeichnet, daß** der Reflektor Strahlung zu einem Zielbereich liefert und die Strahlung Einfallswinkel von 40 bis 180 Grad umfaßt.

7. Reflektor (110, 120, 220) nach einem der Ansprüche 1 bis 6, **dadurch gekennzeichnet, daß** der Reflektor ein Kontrastverhältnis von weniger als 1,5 liefert.

8. Reflektor (110, 120, 220) nach einem der Ansprüche 1 bis 7, umfassend eines oder mehrere der folgenden reflektierende Materialien: Seltene Erden, Seltenerdhalogenide oder Metalloxidverbindungen .

9. Reflektor (110, 120, 220) nach einem der Ansprüche 1 bis 7, umfassend eines oder mehrere der folgenden reflektierenden Materialien: Kalziumoxid, Hafniumoxid, Lanthanoxid, Terbiumoxid, Bariumtitanat, Magnesiumfluorid, Magnesiumoxid, Aluminiumoxid, Bariumoxid, Holmiumoxid, Germaniumoxid, Telluroxid, Europiumoxid, Erbiumoxid, Neodymoxid, Samariumoxid, Ytterbiumoxid, Yttriumoxid, Bariumsulfat oder Dysprosiumoxid, vorzugsweise Magnesiumoxid, Magnesiumfluorid, Aluminiumoxid, Bariumsulfat, Lanthanoxid, Yttriumoxid oder Ytterbiumoxid, am bevorzugtesten Magnesiumoxid, Magnesiumfluorid, Aluminiumoxid oder Bariumsulfat.

10. Reflektor (110, 120, 220) nach einem der Ansprüche 1 bis 9, umfassend ein oder mehrere reflektierende(s) Material(ien), das/die mehr als 99,9 % rein sind.

11. Reflektor (110, 120, 220) nach einem der Ansprüche 1 bis 8, **dadurch gekennzeichnet, daß** der Reflektor eine reflektierende Beschichtung oder Schicht (122, 134, 210) umfaßt, die auf einen Träger (121, 123, 201) aufgebracht ist.

12. Reflektor (110, 120, 220) nach Anspruch 11, **dadurch gekennzeichnet, daß** der Reflektor eine reflektierende Beschichtung (122, 134) umfaßt, die durch Streichen, Sprühen, Tauchen, Gießen, Chromatieren/Phosphatieren, Gelbeschichtung, Ätzen, chemische Dampfabscheidung, Spritzen, Plasmasprühen oder Laserdeposition aufgebracht ist.

13. Reflektor (110, 120, 220) nach Anspruch 11 oder Anspruch 12, **dadurch gekennzeichnet, daß** die reflektierende Beschichtung oder Schicht (122, 134, 210) eine Dicke von 0,1 bis 2500 Mikrometern hat.

14. Reflektor (110, 120, 220) nach einem der Ansprüche 1 bis 8, **dadurch gekennzeichnet, daß** der Reflektor ein verdichtetes Pulver oder einen Block aus reflektierendem Material umfaßt.

15. Reflektor (110, 120, 220) nach einem der Ansprüche 1 bis 8, **dadurch gekennzeichnet, daß** der Reflektor ein Polyvinylalkohol-, Cyanoacrylat-, Acryl- oder Silokonmaterial umfaßt.

16. Reflektor (110, 120, 220) nach einem der Ansprüche 1 bis 8, **dadurch gekennzeichnet, daß** der Reflektor ein Natriumsilikat-, niedrigtemperaturgesintertes Glas- oder Alkalioxidsilikatmaterial umfaßt.

17. Reflektor (110, 120, 220) nach einem der Ansprüche 1 bis 8, **dadurch gekennzeichnet, daß** der Reflektor eines der folgenden Materialien umfaßt: Aluminium-tert-butoxid, Natriumsilikat, Tetraethylorthosilikat (TEOS), Metallisopropoxid, Dysprosiumisopropoxid, Dysprosiumethylhexanodiisopropoxid in Isopropanol, Dysprosium-2-Ethylhexanoat in Hexan, Dysprosiumisopropoxid in Toluol-Isopropanol, Dysprosium-2-Methoxyethoxid in 2-Methoxyethanol, Erbiumethylhexanodiisopropoxid in Isopropanol, Erbium-2-Ethylhexanoat in Hexan, Erbiumisopropoxid in Toluol-Isopropanol, Holmiumethylhexanodiisopropoxid in Isopropanol, Holmiumisopropoxid in Toluol-Isopropanol, Holmium-2-Methoxyethoxid in 2-Methoxyethanol, Lanthanacetat, Lanthan-2-Ethylhexanoat in Hexan, Lanthanisopropoxid, Lanthan-2-Methoxyethoxid in 2-Methoxyethanol, Magnesiumethoxid in Ethanol, Magnesiummethoxid in Methanol, Magnesium-2-Methoxyethoxid in 2-Methoxyethanol, Neodymethylhexanodiisopropoxid in Isopropanol, Neodym-2-Ethylhexanoat in Hexan, Neodymisopropoxid in Toluol-Isopropanol, Neodym-2-Methoxyethoxid in 2-Methoxyethanol, Samariumethylhexanomonoisopropoxid in Toluol-Isopropanol, Samarium-2-Ethylhexanoat in Hexan, Samariumisopropoxid in Toluol-Isopropanol, Samarium-2-Methoxyethoxid in 2-Methoxyethanol, Ytterbiumisopropoxid in Toluol-Isopropanol, Ytterbium-2-Methoxyethoxid in 2-Methoxyethanol, Yttriumethylhexanodiisopropoxid in Toluol-Isopropanol oder Yttriumethylhexanomonoisopropoxid in Toluol-Isopropanol.

18. Strahlungssystem (100, 200), umfassend mindestens eine Hochenergiestrahlungsquelle (130, 140, 240) und mindestens einen Reflektor (110, 120, 220), der mindestens teilweise die mindestens eine Strahlungsquelle (130, 140, 240) umschließt, wobei der mindestens eine Reflektor (110, 120, 220) diffus ist und einen Qualitätsfaktor von mehr als 1,7 hat, wobei der Qualitätsfaktor als das Verhältnis der gesamten Energie gemessen in einem Zielbereich aller Strahlungsquellen und ihrer Reflektoren, die in einem geschlossenen Hohlraum angeordnet sind, geteilt durch die Summe der Energie jeder Lampe und jedes Reflektors, die individuell im Zielbereich oder Volumen eines offenen Hohlraumes gemessen wird, definiert ist,
wobei die diffuse reflektierende Oberfläche eines oder mehrere der folgenden umfaßt: Aluminiumoxid, Bariumsulfat, Bariumtitanat, Bariumoxid, Kalziumoxid, Ceroxid, Dysprosiumoxid, Erbiumoxid, Europiumoxid, Germaniumdioxid, Hafniumoxid, Holmiumoxid, Lanthanoxid, Magnesiumfluorid, Magnesiumoxid, Neodymoxid, Praseodymiumoxid, Samariumoxid, Telluroxid, Terbiumoxid, Titandioxid, Ytterbiumoxid, Yttriumoxid, Zinkoxid.

19. Strahlungssystem (100, 200) nach Anspruch 18, **dadurch gekennzeichnet, daß** die mindestens eine Strahlungsquelle eine Blitzlichtlampe (130, 140, 240) ist.

20. Strahlungssystem (100, 200) nach Anspruch 18 oder Anspruch 19, ferner umfassend mehrere Strahlungsquellen (130, 140), die jeweils mindestens teilweise von mehreren Reflektoren (110, 120) umschlossen sind, wobei die mehreren Reflektoren (110, 120) diffus sind.

21. Strahlungssystem (100, 200) nach einem der Ansprüche 18 bis 20, **dadurch gekennzeichnet, daß** der mindestens eine Reflektor (110, 120, 220) mit einer diffusen reflektierenden Oberfläche mehr als 50 % der Strahlung von 240 bis 280 nm reflektiert.

22. Strahlungssystem (100, 200) nach einem der Ansprüche 18 bis 21, **dadurch gekennzeichnet, daß** der mindestens eine Reflektor (110, 120, 220) die mindestens eine Strahlungsquelle (130, 140, 240) völlig umschließt, um einen geschlossenen Hohlraum zu bilden.

23. Strahlungssystem (100, 200) nach Anspruch 22, **dadurch gekennzeichnet, daß** alle inneren Oberflächen des Hohlraumes diffus reflektierend sind und mehr als 50 % der Strahlung von 240 bis 280 nm reflektieren.

24. Strahlungssystem (100, 200) nach einem der Ansprüche 18 bis 23, **dadurch gekennzeichnet, daß** der mindestens eine Reflektor (110, 120, 220) elliptisch geformt ist.

25. Strahlungssystem (100, 200) nach einem der Ansprüche 18 bis 24, ferner umfassend ein Zielvolumen, wobei das Zielvolumen eine Kontaktlinse in einer Kontaktlinsenpackung umfaßt.

26. Strahlungssystem (100, 200) nach einem der Ansprüche 18 bis 25, **dadurch gekennzeichnet, daß** jede Lampe (130, 140, 240) mindestens einen Reflektor aufweist und daß der mindestens eine Reflektor einen Qualitätsfaktor liefert, der vorzugsweise größer als 2 und am bevorzugtesten größer als 3 ist.

27. Strahlungssystem (100, 200) nach einem der Ansprüche 18 bis 26, **dadurch gekennzeichnet, daß** die Strahlungsmenge, die von einem Reflektor zu einem anderen Reflektor übergehen kann, ohne das Ziel zu passieren, geringer als 50 % ist.

28. Strahlungssystem (100, 200) nach einem der Ansprüche 18 bis 27, ferner umfassend abschwächende Materialien.

29. Strahlungssystem (100, 200) nach einem der Ansprüche 18 bis 28, **dadurch gekennzeichnet, daß** der mindestens eine Reflektor eines der folgenden reflektierenden Materialien umfaßt: Bariumsulfat, Aluminiumoxid, Magnesiumfluorid oder Magnesiumoxid.

## Revendications

1. Réflecteur (110, 120, 220) pour un système de rayonnement haute énergie comprenant une surface réflectrice diffuse qui reflète plus de 50 % du rayonnement de 240 à 280 nm et présente un facteur qualité supérieur à 1,7, le facteur qualité étant défini comme le rapport entre l'énergie totale mesurée dans une zone cible à partir de toutes les sources de rayonnement et leurs réflecteurs configurés dans une cavité fermée divisée par la somme de l'énergie provenant de chaque lampe et du réflecteur mesuré individuellement dans la zone cible ou le volume d'une cavité ouverte, dans lequel ladite surface réflectrice diffuse comprend un ou plusieurs des matériaux en oxyde d'aluminium, sulfate de baryum, titanate de baryum, oxyde de baryum, oxyde de calcium, oxyde de cérium, oxyde de dysprosium, oxyde d'erbium, oxyde d'europium, dioxyde de germanium, oxyde d'hafnium, oxyde d'holmium, oxyde de lanthane, fluorure de magnésium, oxyde de magnésium, oxyde de néodyme, oxyde de praséodyme, oxyde de samarium, oxyde de tellurium, oxyde de terbium, dioxyde de titane, oxyde d'ytterbium, oxyde d'yttrium, oxyde de zinc.

2. Réflecteur (110, 120, 220) selon la revendication 1, dans lequel ledit réflecteur (110, 120, 220) reflète plus de 75 %, de préférence plus de 90 % du rayonnement de 240 à 280 nm, de façon plus préférentielle plus de 90 % du rayonnement de 250 à 270 nm.

3. Réflecteur (110, 120, 220) selon la revendication 1 ou 2, dans lequel ledit réflecteur (110, 120, 220) est elliptique.

4. Réflecteur (110, 120, 220) selon l'une quelconque des revendications 1 à 3, dans lequel ledit réflecteur (110, 120, 220) fournit de l'énergie de rayonnement à une zone cible dans laquelle ladite variation d'énergie est inférieure à 8 mJ/cm².

5. Réflecteur (110, 120, 220) selon l'une quelconque des revendications 1 à 4, dans lequel ledit réflecteur fournit de l'énergie de rayonnement à une zone cible dans laquelle ladite variation d'énergie est inférieure à 15 pourcent, de préférence inférieure à 10 pourcent.

6. Réflecteur (110, 120, 220) selon l'une quelconque des revendications 1 à 5, dans lequel ledit réflecteur fournit le rayonnement à une zone cible, et ledit rayonnement comprend des angles d'incidence compris entre 40 et 180 degrés.

7. Réflecteur (110, 120, 220) selon l'une quelconque des revendications 1 à 6, dans lequel ledit réflecteur offre un rapport de contraste inférieur à 1,5.

8. Réflecteur (110, 120, 220) selon l'une quelconque des revendications 1 à 7, comprenant un ou plusieurs matériaux réflecteurs oxydés à terres rares, d'halogénures à terres rares ou d'une combinaison métallique.

9. Réflecteur (110, 120, 220) selon l'une quelconque des revendications 1 à 7, comprenant un ou plusieurs matériaux réflecteurs en oxyde de calcium, oxyde d'hafnium, oxyde de lanthane, oxyde de terbium, titanate de baryum, fluorure de magnésium, oxyde de magnésium, oxyde d'aluminium, oxyde de baryum, oxyde d'holmium, oxyde de germanium, oxyde de tellurium, oxyde d'europium, oxyde d'erbium, oxyde de néodyme, oxyde de samarium, oxyde d'ytterbium, oxyde d'yttrium, sulfate de baryum ou oxyde de dysprosium, de préférence oxyde de magnésium, fluorure de magnésium, oxyde d'aluminium, sulfate de baryum, oxyde de lanthane, oxyde d'yttrium ou oxyde d'ytterbium, de façon plus préférentielle oxyde de magnésium, fluorure de magnésium, oxyde d'aluminium ou sulfate de baryum.

10. Réflecteur (110, 120, 220) selon l'une quelconque des revendications 1 à 9, comprenant un ou plusieurs matériaux réflecteurs qui sont purs à plus de 99,9 %.

11. Réflecteur (110, 120, 220) selon l'une quelconque des revendications 1 à 8, dans lequel ledit réflecteur comprend un revêtement ou un film réflecteur (122, 134, 210) fixé à un support (121, 123, 201).

12. Réflecteur (110, 120, 220) selon la revendication 11, dans lequel ledit réflecteur comprend un revêtement réflecteur (122, 134) qui est appliqué par peinture, pulvérisation, trempé, coulage, revêtement de conversion, revêtement gélatineux, gravure à l'acide, dépôt de vapeur chimique, pulvérisation cathodique, pulvérisation par plasma ou dépôt laser.

13. Réflecteur (110, 120, 220) selon la revendication 11 ou 12, dans lequel ledit revêtement ou film réflecteur (122, 134, 210) présente une épaisseur comprise entre 0,1 et 2500 microns.

14. Réflecteur (110, 120, 220) selon l'une quelconque des revendications 1 à 8, dans lequel ledit réflecteur comprend une poudre compacte ou un bloc de matériaux réflecteurs.

15. Réflecteur (110, 120, 220) selon l'une quelconque des revendications 1 à 8, dans lequel ledit réflecteur comprend un matériau en polyvinylalcool, en cyanoacrylate, en acrylique ou en silicone.

16. Réflecteur (110, 120, 220) selon l'une quelconque des revendications 1 à 8, dans lequel ledit réflecteur comprend un matériau en silicate de sodium, en verre fritté basse température ou en silicate d'oxyde alcali.

17. Réflecteur (110, 120, 220) selon l'une quelconque des revendications 1 à 8, dans lequel ledit réflecteur comprend un matériau en tert-butoxide d'aluminium, silicate de sodium, tetra-éthylorthosilicate (TEOS), isopropoxide de métal, isopropoxide de dysprosium, éthylhexanodiisopropoxide de dysprosium dans isopropanol, 2-éthylhexanoate de dysprosium dans hexane, isopropoxide de dysprosium dans toluene-isopropanol, 2-méthoxyéthoxide de dysprosium dans 2-méthoxyéthanol, éthylhexanodiisopropoxide d'erbium dans isopropanol, 2-ethylhexanoate d'erbium dans hexane, isopropoxide d'erbium dans toluene-isopropanol, éthylhexanodiisopropoxide d'holmium dans isopropanol, isopropoxide d'holmium dans toluene-isopropanol, 2-méthoxyéthoxide d'holmium dans 2-méthoxyéthanol, acétate de lanthane, 2-éthylhexanoate de lanthane dans hexane, isopropoxide de lanthane, 2-méthoxyéthoxide de lanthane dans 2-méthoxyéthanol, éthoxide de magnésium dans éthanol, méthoxide de magnésium dans méthanol, 2-méthoxyéthoxide de magnésium dans 2-méthoxyéthanol, éthylhexano-diisopropoxide de néodyme dans isopropanol, 2-éthylhexanoate de néodyme dans hexane, isopropoxide de néodyme dans toluene-isopropanol, 2-méthoxyéthoxide de néodyme dans 2-méthoxyéthanol, éthylhexano-monoisopropoxide de samarium dans toluene-isopropanol, 2-éthylhexanoate de samarium dans hexane, isopropoxide de samarium dans toluene-isopropanol, 2-méthoxyéthoxide de samarium dans 2-méthoxyéthanol, isopropoxide d'ytterbium dans toluene-isopropanol, 2-méthoxyéthoxide d'ytterbium dans 2-méthoxyéthanol, éthylhexanodiisopropoxide d'yttrium dans toluene-isopropanol ou éthylhexano-monoisopropoxide d'yttrium dans toluene-isopropanol.

18. Système de rayonnement (100, 200) comprenant au moins une source de rayonnement haute énergie (130, 140, 240) et au moins un réflecteur (110, 120, 220) qui renferme au moins partiellement ladite au moins une source de rayonnement (130, 140, 240) dans laquelle ledit au moins un réflecteur (110, 120, 220) est diffus, et présente un facteur qualité supérieur à 1,7, le facteur qualité étant défini comme le rapport entre l'énergie totale mesurée dans une zone cible à partir de toutes les sources de rayonnement et leurs réflecteurs configurés dans une cavité fermée divisée par la somme de l'énergie provenant de chaque lampe et du réflecteur mesuré individuellement dans la zone cible ou le volume d'une cavité ouverte, dans lequel ladite surface réflectrice diffuse comprend un ou plusieurs des matériaux en hydroxyde d'aluminium, sulfate de baryum, titanate de baryum, oxyde de baryum, oxyde de calcium, oxyde de cérium, oxyde de dysprosium, oxyde d'erbium, oxyde d'europium, dioxyde de germanium, oxyde d'hafnium, oxyde d'holmium, oxyde de lanthane, fluorure de magnésium, oxyde de magnésium, oxyde de néodyme, oxyde de praséodyme, oxyde de samarium, oxyde de tellurium, oxyde de terbium, dioxyde de titane, oxyde d'ytterbium, oxyde d'yttrium, oxyde de zinc.

19. Système de rayonnement (100, 200) selon la revendication 18, dans lequel ladite au moins une source de rayonnement est une lampe-éclair (130, 140, 240).

20. Système de rayonnement (100, 200) selon la revendication 18 ou 19, comprenant en outre de multiples sources de rayonnement (130, 140) qui sont chacune au moins partiellement encadrée par de multiples réflecteurs (110, 120), dans lesquelles les multiples réflecteurs (110, 120) sont diffus.

21. Système de rayonnement (100, 200) selon l'une quelconque des revendications 18 à 20, dans lequel ledit au moins un réflecteur (110, 120, 220) ayant une surface réflectrice diffuse reflète plus de 50 % du rayonnement de 240 à 280 nm.

22. Système de rayonnement (100, 200) selon l'une quelconque des revendications 18 à 21, dans lequel ledit au moins un réflecteur (110, 120, 220) encadre complètement ladite au moins une source de rayonnement (130, 140, 240) pour former une cavité fermée.

23. Système de rayonnement (100, 200) selon la revendication 22, dans lequel toutes les surfaces intérieures de ladite cavité sont diffusément réflectrices et reflètent plus de 50 % du rayonnement de 240 à 280 nm.

24. Système de rayonnement (100, 200) selon l'une quelconque des revendications 18 à 23, dans lequel ledit au moins un réflecteur (110, 120, 220) est de forme elliptique.

25. Système de rayonnement (100, 200) selon l'une quelconque des revendications 18 à 24, comprenant en outre un volume cible, dans lequel ledit volume cible comprend une lentille de contact dans un boîtier de lentille de contact.

26. Système de rayonnement (100, 200) selon l'une quelconque des revendications 18 à 25, dans lequel chaque lampe (130, 140, 240) comprend au moins un réflecteur, et ledit au moins un réflecteur offre un facteur qualité de préférence supérieur à 2 et de façon plus préférentielle supérieur à 3.

27. Système de rayonnement (100, 200) selon l'une quelconque des revendications 18 à 26, dans lequel la quantité de rayonnement pouvant être transmise d'un réflecteur à un autre sans traverser la cible est inférieure à 50 pourcent.

28. Système de rayonnement (100, 200) selon l'une quelconque des revendications 18 à 27 comprenant en outre des matériaux d'atténuation.

29. Système de rayonnement (100, 200) selon l'une quelconque des revendications 18 à 28, dans lequel ledit au moins un réflecteur comprend un matériau réflecteur en sulfate de barium, oxyde d'aluminium, fluorure de magnésium ou oxyde de magnésium.
